Europäisches Patentamt

European Patent Office

Office européen des brevets

(11) Veröffentlichungsnummer: **0 152 836
B1**

(12) **EUROPÄISCHE PATENTSCHRIFT**

(45) Veröffentlichungstag der Patentschrift: **20.06.90**

(51) Int. Cl.⁵: **A 61 K 7/22**

(21) Anmeldenummer: **85101030.6**

(22) Anmeldetag: **01.02.85**

(54) **Mittel zur oralen Hygiene.**

(30) Priorität: **20.02.84 DE 3406005
05.09.84 DE 3432571**

(43) Veröffentlichungstag der Anmeldung:
**28.08.85 Patentblatt 85/35**

(45) Bekanntmachung des Hinweises auf die
Patenterteilung:
**20.06.90 Patentblatt 90/25**

(84) Benannte Vertragsstaaten:
**AT BE CH DE FR GB IT LI LU NL SE**

(56) Entgegenhaltungen:
**EP-A-0 038 868
DE-A-1 943 971
GB-A-1 498 832
US-A-2 909 535**

**CHEMICAL ABSTRACTS, vol. 41, 1947, Spalte
5669a-c, Columbus, Ohio, US; R.L. WAIN et al.:
"Copper fungicides. IX. Investigations with the
exudate from fungus spores"**

(73) Patentinhaber: **Blendax GmbH
Rheinallee 88
D-6500 Mainz 1 (DE)**

(72) Erfinder: **Frosch, Franz, Dr.
Eddersbacher Berg 6
D-6204 Taunusstein (DE)**

Courier Press, Leamington Spa, England.

**Beschreibung**

Die vorliegende Erfindung betrifft die Verwendung von Zinkaspartat und/oder Kupferaspartat zur Herstellung von Mitteln zur oralen Hygiene, insbesondere Zahn- und Mundpflegemitteln, mit einer die Bildung von Zahnbelag vermindernden bzw. hemmenden Wirksamkeit, sowie eine Zahnpasta, die Zinkaspartat und/oder Kupferaspartat enthält.

Die Verwendung von Zinkverbindungen in Mitteln zur oralen Hygiene, insbesondere zum Zweck der Hemmung oder Verminderung der Bildung von Zahnbelag, ist bereits seit langem bekannt und beispielsweise in der NL—OS 71022423 beschrieben.

In dieser Veröffentlichung ist Zinkcitrat als besonders geeignetes Zinksalz hervorgehoben.

Die Verwendung von Zinkcitrat in Kombination mit Alkaliverbindungen bzw. der Einsatz von Ammonium- oder Alkalizinkcitrat in Zahn- und Mundpflegemitteln ist auch bereits aus den US—PSen 4 289 755 und 4 325 939 sowie der DE—OS 3 021 150 bekannt; durch die Verwendung dieser Komplexsalze soll die Löslichkeit des an sich schwer wasserlöslichen Zinkcitrats erhöht werden.

Es ist auch bereits eine Zahnpasta auf dem Markt, die Zinkcitrat als Wirkstoff enthält. Klinische Untersuchungen haben jedoch gezeigt, daß eine einschlägige Wirkung von Zinkcitrat im Doppelblindversuch nicht erzielt werden konnte, vgl. IADR-Abstracts der British Division Nr. 145, Journal of Dental Research, Vol. 59/Special Issue D/Part I (1981), S. 1827.

Es wurde auch bereits vorgeschlagen, andere Zinkverbindungen wie Zinksalicylat, Zinklactat oder Zinkgluconat (EP—OS 74 082), Zinkkomplexverbindungen mit Carboxymethyloxybernsteinsäure (EP—OS 6708) sowie vor allem Zinkacetat (Journal of the American Dental Association, Vol. 27/Nr. 9 (1940), S. 1379 bis 1393) einzusetzen.

Schließlich wurde auch bereits, zur Überwindung der geschilderten Nachteile und der Erhöhung der Wirksamkeit gegen Zahnbelag, angeregt, Zinksalze in Mischungen mit verschiedenen anderen, gegen Zahnbelag wirksamen Stoffen einzusetzen und damit einen synergistischen Effekt zu erzielen.

Hierbei ist insbesondere auf die in der Schweizerischen Monatsschrift für Zahnheilkunde, Band 93 (1983), S. 689 bis 704, und der EP—OS 49 830 beschriebenen Kombinationen von Zinksalzen, insbesondere Zinkfluorid, und Hexetidin in Mitteln zur Plaquehemmung hinzuweisen.

Weiterhin wurde auch eine angeblich synergistische Wirkung zwischen Tetradecylamin und u.a. Zinksalzen in der US—PS 4 146 607 und der EP—OS 11 663 behauptet.

Schließlich beschreibt die GB—OS 2 052 978 noch die Kombination von Zinkverbindungen mit Glycin.

Trotz all dieser vorgeschlagenen vielfältigen Maßnahmen ist es jedoch bisher nicht gelungen, tatsächlich Zinkverbindungen enthaltende Mittel zur oralen Hygiene herzustellen, die stabil sind und gleichzeitig eine ausreichende Wirksamkeit gegen Zahnbelag aufweisen, obwohl die grundsätzliche Eignung von Zinkverbindungen für diesen Zweck unbestritten ist, wie insbesondere aus neueren Arbeiten in Caries Research, Vol. 17 (1983), S. 310 bis 314, Scandinavian Journal of Dental Research, Vol. 9 (1983), S. 169 bis 174, und insbesondere einer Dissertation von J. Afseth (Oslo, 1983) eindeutig hervorgeht.

Es wurde nun gefunden, daß man gegen Zahnbelag äußerst wirksame und stabile Zusammensetzungen zur oralen Hygiene mit einem Gehalt an Zinkverbindungen dann herstellen kann, wenn man als Zinkverbindung Zinkaspartat einsetzt.

Dabei kann des Zinkaspartat als alleinige Zinkverbindung oder im Gemisch mit weiteren Zinkionen liefernden Verbindungen vorliegen; es ist jedoch in jedem Fall zweckmaßig, daß der Gehalt an Zinkaspartat mehr als die Hälfte des eventuellen Gemisches aus Zinkverbindungen beträgt.

Der bevorzugte Gehalt an Zinkaspartat liegt dabei zwischen 0,05 und 5 Gew.-% der Gesamtzusammensetzung des Mittels, vorzugsweise zwischen 0,25 und 2,5 Gew.-%.

Es ist bekannt, daß das Kupfer-Kation eine die Bildung von Zahnbelag hemmende Wirkung ausübt, wenn entsprechende Lösungen von Kupferverbindungen topisch mit den Zähnen in Berührung gebracht werden (AADR-Abstracts 1979, No. 117; Caries Research, Vol. 18, 1984, S. 434—439).

Diese Kupferionen liefernden Verbindungen weisen die Nachteile, wie sie für andere belagshemmende Verbindungen auf Basis antimikrobieller Stoffe wie beispielsweise quaternären Ammoniumverbindungen oder Chlorhexidinsalzen bekannt geworden sind, insbesondere die Verfärbung der Zähne bei Langzeitanwendung, nicht auf.

Ihrer Anwendung in Zahnpasten und anderen, weitere Wirk- und Hilfsstoffe enthaltenden Mitteln stand jedoch bislang entgegen, daß sie einerseits durch verschiedene dieser Stoffe inaktiviert wurden oder nicht bei jedem wünschenswerten pH-Wert ihre Aktivität aufrechterhielten.

In den EP—A 38 867 und 38 868 sind bereits Zahnpasta-Zusammensetzungen beschrieben, die Kupferverbindungen auch in aktiver Form enthalten. Dies wird gewährleistet durch die optimale Auswahl des jeweiligen Poliermittels.

Obwohl diese Zusammensetzungen sich als prinzipiell aktiv gegen Zahnbelag erwiesen haben, ist es doch wünschenswert, in der Auswahl der Poliermittel und des pH-Wertes nicht auf bestimmte Komponenten bzw. Bereiche eingeschränkt zu sein. Es bestand daher das Bedürfnis, ein Kupferionen lieferndes Zahn- und Mundpflegemittel mit belagshemmender Wirkung zu entwickeln, das beliebig einsetzbar ist und durch in solchen Mitteln üblicherweise vorhandene andere Stoffe in seiner Wirkung nicht negativ beeinflußt wird.

Es wurde nun gefunden, daß dies dann möglich ist, wenn man als Kupferionen liefernden Wirkstoff das Kupfersalz der Asparaginsäure, Kupferaspartat, einsetzt. Kupferaspartat ist praktisch mit allen in Zahn- und Mundpflegemitteln zum Einsatz gelangenden Poliermitteln und sonstigen Ingredienzien verträglich; es ist außerdem über einen weiten pH-Bereich von 4 bis 10 einsetzbar, ohne in seiner Aktivität gegen Zahnbelag spürbar beeinträchtigt zu werden.

Das Kupferaspartat kann den erfindungsgemäßen herzustellenden Mitteln als solches zugesetzt werden; es ist jedoch auch möglich, es in situ in den Mitteln selbst durch Reaktion von Asparaginsäure mit löslichen Kupferverbindungen wie Kupfersulfat, Kupferchlorid, etc., herzustellen.

Der bevorzugte Gehalt an Kupferaspartat liegt dabei zwischen 0,05 und 5 Gew.-% der Gesamtzusammensetzung des Mittels, vorzugsweise zwischen 0,25 und 2,5 Gew.-%.

Aus den im folgenden beschriebenen Vergleichsversuchen ergibt sich die überlegene Wirksamkeit sowohl einer Zinkaspartat (0,16 Gew.-%, berechnet auf Zink) als auch einer Kupferaspartat (0,05 Gew.-%, berechnet auf Kupfer) enthaltenden Zahnpasta gegenüber Zahnpasten mit einem Gehalt an Zinkcitrat oder Zinkacetat bzw. einem angeblich synergistischen Gemisch aus Zinkcitrat und Hexetidin oder Kupfersulfat sowie unbehandelten Kontrollgruppen.

Vergleichsversuch A

20 Tage alte Osborne-Mendel-Ratten wurden in 5 Gruppen mit je 16 Versuchstieren aufgeteilt und erhielten die Plaque-Standard-Kost 2000 F.

Zu Versuchsbeginn wird von jedem Versuchstier der Plaque-Ausgangs-Status erstellt. Anschließend werden die Tiere mit 0,1 ml einer standardisierten Bakteriensuspension von Actinomyces viscosus OMZ 105 geimpft. Eine Nachimpfung erfolgt jeweils in einwöchigem Abstand.

Die Behandlung der Versuchstiere mit den zu untersuchenden Präparaten beginnt am 23. Lebenstag, je zweimal täglich werden mit einer Spritze 0,1 ml Testpräparat/Versuchstier appliziert. Nach 5 Wochen wurden die Tiere getötet und die Plaquebildung an den beiden ersten Bukkalflächen und den ersten 4 Lingualflächen der Molaren 1 und 2 im Oberkiefer beurteilt; d.h., 12 Begutachtungsflächen/Tier.

Die Bewertung erfolgt nach Anfärbung mit Erythrosin-Lösung nach dem folgenden Schema:

0: Keine Plaque
1: Bis zu 1/3 der Fläche mit Plaque überzogen.
2: Bis zu 2/3 der Fläche mit Plaque überzogen.
3: Mehr als 2/3 der Fläche mit Plaque überzogen.
Die Punktzahl wird addiert und ein Mittelwert ($\bar{x}$) gebildet.

Ergebnis

| Gruppe | Sx | $\bar{x}$ | σ |
|--------|-----|-------|------|
| 1 | 227 | 14,19 | 4,56 |
| 2 | 256 | 16,00 | 4,72 |
| 3 | 165 | 10,31 | 3,28 |
| 4 | 249 | 15,56 | 4,03 |
| 5 | 342 | 21,38 | 2,70 |

Beispiel A

| | |
|---|---|
| α-Aluminiumoxidtrihydrat (Korngrößenverteilung etwa 1—15 μm) | 58,5 (Gew.%) |
| Sorbitlösung (70%) | 25,5 |
| Xanthum-Gum | 0,6 |
| Natriummonofluorphosphat | 0,8 |
| Saccharin-Natrium | 0,1 |
| Konservierungsmittel | 0,3 |
| Natriumlaurylsulfat | 0,4 |
| Aromagemisch | 0,1 |
| Zinkaspartat | 0,5 |
| Wasser | ad 100,0 |

Zusammensetzung der Zahnpasten

Gruppe 1: Entsprechend Beispiel A, jedoch anstelle von Zinkaspartat 0,05% Hexetidin und 0,5% Zinkcitrat; 2 $H_2O$ (= 0,16% Zn).

Gruppe 2: Entsprechend Beispiel A, jedoch anstelle von Zinkaspartat 0,5% Zinkcitrat; 2 $H_2O$ (= 0,16% Zn).

Gruppe 3: Erfindungsgemäße Zusammensetzung nach Beispiel A.

Gruppe 4: Entsprechend Beispiel A, jedoch anstelle von Zinkaspartat 0,55% Zinkacetat 2 $H_2O$ (= 0,16% Zn).

Gruppe 5: Unbehandelte Kontrolle.

Der Gewichtsausgleich auf 100% erfolgte jeweils durch Variation des Wassergehaltes.

Das Ergebnis zeigt die überraschende Überlegenheit des Zinkaspartats, sogar gegenüber einer im Stand der Technik als "synergistisches Gemisch" bezeichneten Zusammensetzung mit einem Gehalt an Zinkverbindungen und Hexetidin.

Vergleichsversuch B

Dieser wurde entsprechend der bei Vergleichsversuch A beschriebenen Verfahrensweise durchgeführt.

Ergebnis

| Gruppe | $\bar{x}$ |
|--------|-----------|
| 1 | 10,31 ± 3,42 |
| 2 | 10,75 ± 3,26 |
| 3 | 8,44 ± 2,06 |
| 4 | 20,50 ± 3,32 |
| 5 | 23,94 ± 2,43 |

Beispiel A

| | |
|---|---|
| α-Aluminiumoxidtrihydrat (Korngrößenverteilung etwa 1—15 μm) | 58,50 (Gew.%) |
| Sorbitlösung (70%) | 25,50 |
| Xanthum-Gum | 0,60 |
| Natriummonofluorphosphat | 0,80 |
| Saccharin-Natrium | 0,10 |
| Konservierungsmittel | 0,30 |
| Natriumlaurylsulfat | 0,40 |
| Aromagemisch | 0,10 |
| Kupferaspartat | 0,26 |
| Wasser | ad 100,00 |

### Beispiel B

| | |
|---|---|
| Synthetisches Zeolith A $(Na_{12}(AlO_2)_{12}(SiO_2)_{12}27\ H_2O)$ | 24,00 (Gew.%) |
| Dicalciumorthophosphat | 10,00 |
| Carboxymethylcellulose | 1,20 |
| Natriumlaurylsulfat | 2,00 |
| Glycerin | 6,00 |
| Sorbit | 15,00 |
| Konservierungsmittel | 0,30 |
| Aromagemisch | 1,00 |
| Pyrogene Kieselsäure | 1,55 |
| Saccharin-Natrium | 0,05 |
| Natriummonofluorphosphat | 0,80 |
| Kupferaspartat | 0,25 |
| Wasser | ad 100,00 |

### Beispiel C

| | |
|---|---|
| Calciumcarbonat | 41,00 (Gew.%) |
| Natriumlaurylsulfat | 1,50 |
| Hydroxymethylcellulose | 1,30 |
| Sorbit | 9,00 |
| Natriummonofluorphosphat | 0,80 |
| Aromagemisch | 1,00 |
| Saccharin-Natrium | 0,10 |
| Konservierungsmittel | 0,25 |
| Kupferaspartat | 0,25 |
| Wasser | ad 100,00 |

Zusammensetzung der Zahnpasten

Gruppe 1: Entsprechend Beispiel A.
Gruppe 2: Entsprechend Beispiel B.
Gruppe 3: Entsprechend Beispiel C.
Gruppe 4: Entsprechend Beispiel C, jedoch anstelle von Kupferaspartat 0,20% Kupfersulfat·5H₂O (= 0,05% Cu).
Gruppe 5: Unbehandelte Kontrolle.

Der Gewichtsausgleich auf 100% in Gruppe 4 erfolgte durch Variation des Wassergehaltes.

Das Ergebnis zeigt die überraschende Überlegenheit des Kupferaspartates gegenüber einer Kupfersulfat in gleicher Cu-Konzentration enthaltenden Zusammensetzung, sogar im alkalischen pH-Bereich.

Die erfindungsgemäßen herzustellenden Mittel zur oralen Hygiene können in jeder beliebigen Applikationsform vorliegen. Bevorzugt werden die Applikationsformen Zahnpasta, wobei es sich um eine opake oder um eine gelförmige transparente Zahnpasta handeln kann, Mundwasser und Kaugummi; jedoch ist auch jede andere Applikationsform wie beispielsweise Mundspray, Lutsch- oder Kautablette oder Zahnpulver für diesen Zweck geeignet.

Eine Zahnpasta kann opak oder eine durch Verwendung geeigneter, in ihren Brechungsindices mit dem Brechungsindex des Trägermaterials übereinstimmender Poliermittel hergestellte transparente Zahnpasta sein.

Besonders geeignet als Poliermittel sind Aluminiumoxid, insbesondere in Form seines Trihydrates wie α-Aluminiumoxidtrihydrat, mit einer bevorzugten Korngrößenverteilung zwischen 1 und 20, vorzugsweise etwa 10 μm, und Calciumcarbonat. Es ist jedoch auch möglich, Zahnpasten auf anderer Pastengrundlage einzusetzen, die als Poliermittel beispielsweise Alkaliuminiumsilikate wie solche vom Zeolith-Typ A, beschrieben in den EP-PSen Nr. 2690 und 3023, verschiedene Calciumphosphate wie Dicalciumorthophosphat in Form seines Dihydrats oder wasserfrei, Tricalciumphosphat, Calciumpyrophosphat, unlösliche Alkalimetaphosphate, Siliciumdioxide verschiedener Modifikationen wie Siliciumdioxid-Xerogele, -Hydrogele oder gefällte Siliciumdioxide, oder pulverförmige Kunststoffe wie Polymethylmethacrylat mit einer Korngrößenverteilung zwischen 0,5 und 5 μm enthalten.

Es können selbstverständlich auch Poliermittelgemische aus den genannten Substanzen eingesetzt werden, beispielsweise ein Gemisch aus α-Aluminiumoxidhydrat und/oder Calciumcarbonat und synthetischem Zeolith A im Verhältnis von etwa 1:1.

Der Poliermittelanteil in den erfindungsgemäßen Zahnpasten liegt vorzugsweise zwischen 20 und 60 Gew.-% der Gesamtzusammensetzung.

Besonders geeignet sind erfindungsgemäße Zahnpasten, bei denen das Poliermittel zum überwiegenden Teil (mehr als 50 Gew.-%) entweder aus Aluminiumoxidhydrat oder aus Calciumcarbonat besteht.

Es ist selbstverständlich möglich, die üblichen in Zahnpasten eingesetzten oberflächenaktiven Verbindungen in Mengen bis zu etwa 2,5 Gew.-% der Gesamtzusammensetzung zu verwenden.

Solche synthetischen oberflächenaktiven Stoffe sind beispielsweise Alkylsulfate, Alkylethersulfate, Olefinsulfonate, Natriumlauroylsarcosinat oder ampholytische, nichtionische oder kationaktive Verbindungen oder auch Seifen wei beispielsweise solche von Laurinsäure, Myristinsäure, Palmitinsäure, Stearinsäure oder Gemischen derselben, beispielsweise Cocosölfettsäuren oder Talgfettsäuren.

Eine Übersicht über in Zahnpasten einsetzbare Zusammensetzungen findet sich, wie überhaupt über sonstige zur Herstellung von Zahnpflegemitteln üblicherweise zum Einsatz gelangende Stoffe und die dabei angewandten Herstellungsverfahren, in dem Handbuch von M. S. Balsam und E. Sagarin, "Cosmetics — Science and Technology", 2nd Ed., Vol. 1, S. 423 bis 533 (1972), auf das hier ausdrücklich Bezug genommen wird.

Gleiches gilt hinsichtlich der in Zahnpasten üblicherweise in Mengen zwischen 10 und 35 Gew.-% zum Einsatz gelangenden Feuchthaltemittel, wie Glycerin, Diole wie 1,4-Butandiol oder 1,2-Propandiol oder Zuckeralkohole wie Sorbit, Mannit oder Xylit und Polyglykole mit niederen Molekulargewichten, ebenso für Verdickungsmittel, deren Mengenanteil in Zahnpasten zwischen 0,25 und 5 Gew.-% der Gesamtzusammensetzung liegt.

Bevorzugte Verdickungsmittel sind Carboxymethylcellulose und deren Alkalisalze, insbesondere Natriumcarboxymethylcellulose, Hydroxyalkylcellulosen wie Hydroxymethylcellulose und Hydroxyethylcellulose, Methylcellulose, Pflanzengummen wie Tragant, Gummi arabicum, Carayagummi, Guargummi, Xanthangummi und Irish Moos, synthetische Polyelektrolyte wie die Alkalisalze der Polyacrylsäure sowie anorganische Verdickungsmittel, beispielsweise kolloidales Magnesiumaluminiumsilikat oder disperses Siliciumoxid.

In den erfindungsgemäßen Miteln zur oralen Hygiene können selbstverständlich auch weitere Wirkstoffe Verwendung finden. Solche sind insbesondere die bekannten kariesprophylaktischen Fluoride, vorzugsweise in einer solchen Menge, daß die Konzentration an reinem Fluor im Mittel 0,05 bis 1 Gew.-%, vorzugsweise 0,1 bis 0,5 Gew.-% des Mittels beträgt.

Geeignete Fluorverbindungen sind insbesondere die verschiedenen Salze der Monofluorphosphorsäure wie Natrium-, Kalium-, Lithium-, Calcium- und Aluminiummono- und difluorphosphat sowie verschiedene, Fluor in ionisch gebundener Form enthaltende Fluoride, insbesondere Alkalifluoride wie Natrium-, Lithium-, Kalium- und Ammoniumfluorid, Zinnfluorid, Manganfluorid, Kupferfluorid, Zirkoniumfluorid und Aluminiumfluorid sowie Gemische oder Anlagerungsprodukte dieser Fluoride untereinander und mit anderen Fluorverbindungen, beispielsweise Alkalimanganfluoride.

Weitere, in den erfindungsgemäßen Mitteln zur oralen Hygiene einsetzbare Stoffe sind Zahnbelag entfernende Substanzen, beispielsweise Zinksalze, Mittel zur Verhinderung von Zahnsteinbildung wie Hydroxyethan-1,1-diphosphonsäure oder Alkylendiaminotetramethylenphosphonsäuren und deren wasserlösliche Salze, Allantoin, Azulen, etc.

Im folgenden werden einige Beispiele gegeben, die das Wesen der vorliegenden Erfindung charakterisieren:

EP 0 152 836 B1

Zahnpasten

| Komponenten | Beispiel Nr. | | | |
|---|---|---|---|---|
| | 1 | 2 | 3 | 4 |
| α-Aluminiumhydroxid (Gew.-%) | 35,0 - 60,0 | | | |
| Pyrogenes Siliciumdioxid | 0,5 - 4,0 | 0,5 - 4,0 | 0,5 - 4,0 | 0,5 - 4,0 |
| Natriumlaurylsulfat | 1,0 - 2,0 | 1,0 - 2,0 | 1,0 - 2,0 | 1,0 - 2,0 |
| Sorbit | 5,0 - 30,0 | 5,0 - 80,0 | 5,0 - 30,0 | 5,0 - 30,0 |
| Glycerin | 5,0 - 30,0 | 5,0 - 30,0 | 5,0 - 30,0 | 5,0 - 30,0 |
| Bindemittel (Xanthum-Gum, Carboxymethylcellulose) | 0,5 - 2,0 | 0,5 - 2,0 | 0,5 - 2,0 | 0,5 - 2,0 |
| Konservierungsmittel | 0,1 - 0,3 | 0,1 - 0,3 | 0,1 - 0,3 | 0,1 - 0,3 |
| Saccharin-Natrium oder Natriumcyclamat | 0,1 - 0,3 | 0,1 - 0,3 | 0,1 - 0,3 | 0,1 - 0,3 |
| Aromagemisch | 0,8 - 1,5 | 0,8 - 1,5 | 0,8 - 1,5 | 0,8 - 1,5 |
| Natriummonofluorphosphat | 0,5 - 1,2 | 0,5 - 1,2 | 0,5 - 1,2 | 0,5 - 1,2 |
| Natriumfluorid | 0,05 - 0,2 | 0,05 - 0,2 | - - | 0,05 - 0,2 |
| Zinkaspartat | 0,25 - 2,5 | 0,25 - 2,5 | 0,25 - 2,5 | 0,25 - 2,5 |
| Siliciumdioxid-Xerogel ( 2 - 15 µm; 700 m²/g) | | 15,0 - 25,0 | | |
| Gefälltes Calciumcarbonat | | | 30,0 - 50,0 | |
| Polymethylmethacrylat-Pulver | | | | 35,0 - 55,0 |
| Wasser | ad 100,0 | ad 100,0 | ad 100,0 | ad 100,0 |

Zahnpasten

| Komponenten | Beispiel Nr. | | | |
|---|---|---|---|---|
| | 5 | 6 | 7 | 8 |
| $\alpha$-Aluminiumhydroxid (Gew.%) | 35,0 - 60,0 | | | |
| Pyrogenes Siliciumdioxid | 0,5 - 4,0 | 0,5 - 4,0 | 0,5 - 4,0 | 0,5 - 4,0 |
| Natriumlaurylsulfat | 1,0 - 2,0 | 1,0 - 2,0 | 1,0 - 2,0 | 1,0 - 2,0 |
| Sorbit | 5,0 - 30,0 | 5,0 - 80,0 | 5,0 - 30,0 | 5,0 - 30,0 |
| Glycerin | 5,0 - 30,0 | 5,0 - 30,0 | 5,0 - 30,0 | 5,0 - 30,0 |
| Bindemittel (Xanthum-Gum, Carboxymethylcellulose) | 0,5 - 2,0 | 0,5 - 2,0 | 0,5 - 2,0 | 0,5 - 2,0 |
| Konservierungsmittel | 0,1 - 0,3 | 0,1 - 0,3 | 0,1 - 0,3 | 0,1 - 0,3 |
| Saccharin-Natrium oder Natriumcyclamat | 0,1 - 0,3 | 0,1 - 0,3 | 0,1 - 0,3 | 0,1 - 0,3 |
| Aromagemisch | 0,8 - 1,5 | 0,8 - 1,5 | 0,8 - 1,5 | 0,8 - 1,5 |
| Natriummonofluorphosphat | 0,5 - 1,2 | 0,5 - 1,2 | 0,5 - 1,2 | 0,5 - 1,2 |
| Natriumfluorid | 0,05 - 0,2 | 0,05 - 0,2 | - | 0,05 - 0,2 |
| Kupferaspartat | 0,25 - 2,5 | 0,25 - 2,5 | 0,25 - 2,5 | 0,25 - 2,5 |
| Siliciumdioxid-Xerogel ( 2 - 15 µm; 700 m²/g) | | 15,0 - 25,0 | | |
| Gefälltes Calciumcarbonat | | | 30,0 - 50,0 | |
| Dicalciumorthophosphat | | | | 35,0 - 55,0 |
| Wasser | ad 100,0 | ad 100,0 | ad 100,0 | ad 100,0 |

EP 0 152 836 B1

Beispiel 9

*Mundwasser-Konzentrat:*

| | |
|---|---|
| Aromagemisch | 5,00 (Gew.%) |
| Zinkaspartat | 3,30 |
| Zinkcitrat·2 $H_2O$ | 0,25 |
| Nichtionischer Emulgator | 1,80 |
| n-Propanol | 5,00 |
| 1-Methoxypropanol | 35,00 |
| Glycerin | 8,50 |
| Phenylsalicylat | 0,55 |
| Saccharin-Natrium | 0,30 |
| Wasser | 40,30 |

Das Konzentrat wird vor Gebrauch im Verhältnis von etwa 1:4 mit Wasser verdünnt.

Beispiel 10

*Kaugummi:*

| | |
|---|---|
| Gummibase | 30,00 |
| Sorbit | 25,00 |
| Xylit | 20,00 |
| Saccharin-Natrium | 0,30 |
| Zinkacetat·2 $H_2O$ | 0,30 |
| Zinkaspartat | 2,40 |
| Kupfersulfat·2 $H_2O$ | 0,30 |
| Glycerin | 2,00 |
| Aromagemisch | 3,70 |
| Ascorbinsäure | 1,00 |
| Fructose | 15,00 |

Beispiel 11

*Mundwasser-Konzentrat:*

| | |
|---|---|
| Aromagemisch | 5,00 (Gew.%) |
| Kupferaspartat | 2,50 |
| Zinkcitrat·2 $H_2O$ | 0,25 |
| Nichtionischer Emulgator | 1,80 |
| n-Propanol | 5,00 |

9

# EP 0 152 836 B1

| | |
|---|---|
| 1-Methoxypropanol (−2) | 35,00 |
| Glycerin | 8,50 |
| Phenylsalicylat | 0,55 |
| Saccharin-Natrium | 0,30 |
| Wasser | ad 100,00 |

Das Konzentrat wird vor Gebrauch im Verhältnis von etwa 1:4 mit Wasser verdünnt.

## Beispiel 12

*Kaugummi:*

| | |
|---|---|
| Gummibase | 30,00 |
| Sorbit | 25,00 |
| Xylit | 20,00 |
| Saccharin-Natrium | 0,30 |
| Kupferfluorid | 0,50 |
| Kupferaspartat | 2,20 |
| Kupfersulfat·2 H$_2$O | 0,30 |
| Glycerin | 2,00 |
| Aromagemisch | 3,70 |
| Ascorbinsäure | 1,00 |
| Fructose | 15,00 |

## Patentansprüche

1. Verwendung von Zinkaspartat und/oder Kupferaspartat zur Herstellung eines Mittels zur oralen Hygiene zur Verminderung bzw. Hemmung der Bildung von Zahnbelag.

2. Verwendung nach Anspruch 1, dadurch gekennzeichnet, daß dem Mittel 0,05 bis 5 Gew.-%, berechnet auf die Gesamtzusammensetzung, Zinkaspartat oder Kupferaspartat zugesetzt werden.

3. Verwendung nach Anspruch 2, dadurch gekennzeichnet, daß dem Mittel 0,25 bis 2,5 Gew.-%, berechnet auf die Gesamtzusammensetzung, Zinkaspartat oder Kupferaspartat zugesetzt werden.

4. Zahnpasta auf wäßriger Basis, enthaltend ein Poliermittel, dadurch gekennzeichnet, daß sie Zinkaspartat und/oder Kupferaspartat enthält.

5. Zahnpasta nach Anspruch 4, dadurch gekennzeichnet, daß das Poliermittel zum überwiegenden Teil (mehr als 50 Gew.-%) aus Aluminiumoxidhydrat besteht.

6. Zahnpasta nach Anspruch 4, dadurch gekennzeichnet, daß das Poliermittel zum überwiegenden Teil (mehr als 50 Gew.-%) aus Calciumcarbonat besteht.

## Revendications

1. Utilisation de l'aspartate de zinc et/ou de l'aspartate de cuivre pour préparer un produit pour l'hygiène buccale, pour diminuer ou inhiber la formation du tartre dentaire.

2. Utilisation selon la revendication 1, caractérisée en ce que l'on ajoute au produit 0,05 à 5% en poids, pourcentage calculé sur la composition totale, d'aspartate de zinc ou d'aspartate de cuivre.

3. Utilisation selon la revendication 2, caractérisée en ce que l'on ajoute au produit 0,25 à 2,5% en poids, pourcentage calculé sur la composition totale, d'aspartate de zinc ou d'aspartate de cuivre.

4. Pâte dentifrice à base aqueuse, contenant un agent de polissage, pâte caractérisée en ce qu'elle contient de l'aspartate de zinc et/ou de l'aspartate de cuivre.

5. Pâte dentifrice selon la revendication 4, caractérisée en ce que l'agent de polissage consiste pour une partie prédominante (plus de 50% en poids) en oxyde d'aluminium hydraté.

## EP 0 152 836 B1

6. Pâte dentifrice selon la revendication 4, caractérisée en ce que l'agent de polissage consiste pour une partie prédominante (plus de 50% en poids) en carbonate de calcium.

### Claims

1. Use of zinc aspartate and/or copper aspartate for the preparation of an agent for oral hygiene to reduce or inhibit the formation of dental plaque.

2. Use according to Claim 1, characterized in that 0.05 to 5% by weight, calculated on the basis of the total composition, of zinc aspartate or copper aspartate are added to the agent.

3. Use according to Claim 2, characterized in that 0.25 to 2.5% by weight, calculated on the basis of the total composition, of zinc aspartate or copper aspartate are added to the agent.

4. Water-based toothpaste containing a polishing agent, characterized in that it contains zinc aspartate and/or copper aspartate.

5. Toothpaste according to Claim 4, characterized in that the polishing agent consists predominantly (more than 50% by weight) of aluminium oxide hydrate.

6. Toothpaste according to Claim 4, characterized in that the polishing agent consists predominantly (more than 50% by weight) of calcium carbonate.